# EUROPÄISCHE PATENTANMELDUNG

(11) **EP 2 439 189 A1**
(43) Veröffentlichungstag der Anmeldung: **11.04.2012**
(21) Anmeldenummer: 10177390.1
(22) Anmeldetag: 17.09.2010
(51) Int. Cl.: C07C 209/18, C07C 211/45, B01J 23/44, B01J 35/08

(54) **Verfahren zur Herstellung von aromatischen Aminen**

(71) Anmelder: BASF SE, 67056 Ludwigshafen (DE)
(72) Erfinder: Ahrens, Sebastian, 69168, Wiesloch (DE); Müller, Christoph, 68165, Mannheim (DE); Paul, Axel, 68623, Lampertheim (DE); Ernst, Martin, 69121, Heidelberg (DE); Pollmer, Nadja, 67281, Bissersheim (DE); Melder, Johann-Peter, 67459, Böhl-Iggelheim (DE); Heidemann, Thomas, 68519, Viernheim (DE); Anders, Joachim-Thierry, 01328, Dresden (DE); Hoffer, Bram Willem, Fanwood, NJ 07023 (US)

(57) **Zusammenfassung**

Die vorliegende Erfindung betrifft ein Verfahren zur Herstellung eines aromatischen Amins durch Umsetzung eines entsprechenden aromatischen Alkohols mit einem Aminierungsmittel ausgewählt aus der Gruppe bestehend aus Ammoniak, primärer Amine und sekundärer Amine, in Gegenwart von Wasserstoff und eines Katalysatorformkörpers, bei einer Temperatur von 60 bis 300°, dadurch gekennzeichnet, dass der Katalysatorformkörper Zr, Pd und Pt enthält und eine Ringtablettenform mit einem Außendurchmesser im Bereich von 2 bis 6 mm und einer Höhe im Bereich von 1 bis 4 mm und einem Innendurchmesser von 1 bis 5 mm oder eine topologisch äquivalente Form mit gleichem Volumen aufweist.

Ein weiterer Gegenstand der vorliegenden Erfindung sind Katalysatorformkörper enthaltend Zr, Pd und Pt, dadurch gekennzeichnet dass der Katalysatorformkörper eine Ringtablettenform, mit einem Außendurchmesser im Bereich von 3 bis 6 mm und einer Höhe im Bereich von 1 bis 4 mm und einem Innendurchmesser von 2 bis 5 mm oder eine topologisch äquivalente Form aufweist.

## Beschreibung

Die vorliegende Erfindung betrifft ein Verfahren zur Herstellung eines aromatischen Amins durch Umsetzung eines entsprechenden aromatischen Alkohols mit einem Aminierungsmittel in Gegenwart von Wasserstoff und einem Katalysatorformkörper, der eine spezielle Ringtablettenform oder eine topologisch äquivalente Form aufweist. Weiterhin betrifft die vorliegende Erfindung Katalysatorformkörper mit einer speziellen Ringtablettenform oder einer topologisch äquivalenten Form.

Aromatische Amine, wie 2,6-Xylidin, finden u.a. Verwendung als Zwischenprodukte bei der Herstellung von Kraftstoffadditiven (US-A-3,275,554; DE-A-21 25039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, lonenaustauschern, Textilhilfsmitteln, Farbstoffen, Vulkanisationsbeschleunigern und/oder Emulgatoren.

Aus der US-A-5,072,044 ist die Dehydrierung von Cyclohexylaminen zu den entsprechenden aromatischen Aminen an Li-dotierten Pd-Katalysatoren bekannt. Die Umsetzung wird bei hohen Reaktionstemperaturen von 360-380 °C durchgeführt.

In der EP-A-701 995 ist ein Verfahren beschrieben zur Herstellung von aromatischen Aminen aus den entsprechenden cycloaliphatischen Aminen in Gegenwart von Wasserstoff und Ammoniak und an einem bimetallischem Palladium-/Platin-Katalysator. Das cycloaliphatische Amin muss hierfür in einem anderen Prozess erst zur Verfügung gestellt werden. Es werden hier unter anderem Pd/Pt-ZrO₂-Katalysatoren in Form von Strängen für die Umsetzung von Dimethylcyclohexylamin (DMCHA) zu 2,6-Xylidin beschrieben.

Die EP-A-22751 beschreibt die Umsetzung von Phenolen in Gegenwart von Ammoniak und Wasserstoff zu den entsprechenden Cyclohexylaminen in Gegenwart von Edelmetallkatalysatoren mit Tonerde oder Kohle als Trägermaterial.

In der EP-A-53 819 wird ein Verfahren zur Herstellung von cycloaliphatischen und/oder aromatischen Aminen aus Phenolen offenbart. Das eingesetzte Katalysatorsystem enthält Ru, Rh oder Pt auf einem Aluminiumoxid-Träger.

Die EP-A-167 996 beschreibt ein Verfahren zur Herstellung von aromatischen Aminen durch Umsetzung des entsprechenden Phenols ggf. in Gegenwart des entsprechenden zurückgeführten cycloaliphatischen Amins in Gegenwart von Ammoniak und Wasserstoff an einem Edelmetall-Katalysator bei Normaldruck und in zwei hintereinandergeschalteten Reaktionszonen. Bevorzugt sind Aluminiumoxid-geträgerte Katalysatoren.

CA Abstract No. 132:336078 (CN-B-1 087970) betrifft die Synthese von 2,6-Dimethylanilin aus 2,6-Dimethylphenol bei 180-200 °C an einem spezifischen Pd/Al₂O₃-Mg/Al₂O₃ Katalysator.

In der WO 2006/136573 wird ein Verfahren zur kontinuierlichen Herstellung von primären aromatischen Aminen durch Umsetzung der entsprechenden aromatischen Alkohole mit Ammoniak in Gegenwart von Wasserstoff an Heterogenkatalysatoren offenbart, wobei der Heterogenkatalysator Palladium, Platin und Zirkonium enthält.

Die WO 2007/077148 beschreibt ein Verfahren zur kontinuierlichen Herstellung eines primären aromatischen Amins durch Umsetzung eines entsprechenden cycloaliphatischen Alkohols mit Ammoniak in Gegenwart eines Heterogenkatalysators, dadurch gekennzeichnet, dass die katalytisch aktive Masse des Katalysators vor dessen Reduktion mit Wasserstoff Palladium, Platin und Zirkonium enthält.

Der vorliegenden Erfindung lag die Aufgabe zugrunde, ein verbessertes wirtschaftliches Verfahren zur Herstellung von primären aromatischen Aminen aus Phenolen aufzufinden. Insbesondere soll das Verfahren bessere Ausbeuten, Raum-Zeit-Ausbeuten (RZA) und Selektivitäten ermöglichen. Darüber hinaus sollte die Bildung von typischen Nebenprodukten, wie Cyclohexanolen, Dicyclohexylamin, Diphenylaminen u.a., die nur schwer vom Wertprodukt abzutrennen sind, verringert werden. Des Weiteren war es ein Ziel der vorliegenden Erfindung einen Katalysator mit einer höheren Aktivität zur Verfügung zu stellen, der seine Aktivität über einen möglichst langen Zeitraum aufrecht erhält. Der verwendete Katalysator sollte somit verbesserte Standzeiten aufweisen und weniger häufig regeneriert werden müssen, um die Abstellungen zum Katalysatorwechsel im technischen Betrieb verringern zu können.

Die Aufgabe der vorliegenden Erfindung wurde gelöst, durch ein Verfahren zur Herstellung eines aromatischen Amins durch Umsetzung eines entsprechenden aromatischen Alkohols mit einem Aminierungsmittel ausgewählt aus der Gruppe bestehend aus Ammoniak, primärer Amine und sekundärer Amine,
in Gegenwart von Wasserstoff und
eines Katalysatorformkörpers,
bei einer Temperatur von 60 bis 300°,
dadurch gekennzeichnet, dass der Katalysatorformkörper Zr, Pd und Pt enthält und eine Ringtablettenform mit einem Außendurchmesser im Bereich von 2 bis 6 mm und einer Höhe im Bereich von 1 bis 4 mm und einem Innendurchmesser von 1 bis 5 mm oder eine topologisch äquivalente Form mit gleichem Volumen aufweist.

In dem erfindungsgemäßen Verfahren wird ein aromatischer Alkohol eingesetzt, der mit einem Aminierungsmittel in Gegenwart von Wasserstoff und einem Katalysatorformkörper zu einem entsprechenendem aromatischen Amin umgesetzt wird.

Als aromatische Alkohole können praktisch alle Alkohole mit aromatischer OH-Funktion eingesetzt werden, in denen eine OH-Gruppe an einem sp²-hybridisierten C-Atom des aromatischen Rings gebunden ist. Der aromatische Ring kann neben C-Atomen auch ein oder mehrere Heteroatome, wie N, O oder S, aufweisen. Die Alkohole können ferner Substituenten tragen oder funktionelle Gruppen enthalten, welche sich unter den Bedingungen der hydrierenden Aminierung inert verhalten, beispielsweise Alkyl-, Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige aromatische Alkohole aminiert werden, so hat man es über die Steuerung der Reaktionsbedingungen in der Hand, bevorzugt entsprechende Aminoalkohole oder mehrfach aminierte Produkte zu erhalten.

Beispiele für aromatische Alkohole, die in das erfindungsgemäße Verfahren eingesetzt werden können sind ortho-, meta- und para-Kresol, ortho-Ethylphenol, ortho-n-Butylphenol, ortho-sec.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-cyclohexyl-phenol, 2,6-Diethylphenol, 2,5-Diisopropylphenol, 2-Methyl-6-sec.-butylphenol, 3-tert.-Butylphenol, 2,6-Diisopropylphenol, 2,6-Di-sec.-Butylphenol, 2,6-Dicyclohexylphenol, alpha-Naphthol, beta-Naphthol, Bisphenol A (= 2,2-Di(p-hydroxyphenyl)propan, Hydrochinon, Mono-, Di-, Tri- oder Tetraalkylhydrochinone, insbesondere mit C₁-₉-Alkylresten (unabhängig voneinander) substituierte Hydrochinone, z.B. Monomethylhydrochinon, Tetramethylhydrochinon.

Bevorzugt werden aromatische Alkohole der Formel (I) eingesetzt 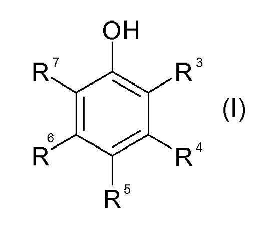 in der R³,R⁴, R⁵, R⁶ und R⁷ jeweils zusammen oder unabhängig voneinander

Wasserstoff,
C₁-C₁₂-Alkyl, bevorzugt C₁- bis C₈-Alkyl, besonders bevorzugt C₁- bis C₄-Alkyl, wie Methyl, Ethyl, n-Propyl, iso- Propyl, n-Butyl, iso-Butyl, sec.-Butyl und tert.-Butyl, oder
C₃-C₁₂-Cycloalkyl, bevorzugt C₅- bis C₈-Cycloalkyl, besonders bevorzugt C₅- bis C₆-Cycloalkyl, wie Cyclopentyl und Cyclohexyl, bedeuten.

Besonders bevorzugt werden alkylsubstituierte Phenole in das erfindungsgemäße Verfahren eingesetzt, wobei die Substituenten bevorzugt C₁-₁₂-alkyl, besonders bevorzugt C₁-₉-alkyl und ganz besonders bevorzugt C₁-₄-alkyl sind.

Es ist weiterhin bevorzugt, dass 1 bis 3, bevorzugt 1 bis 2, der Substituenten R³,R⁴, R⁵, R⁶ und R⁷ entsprechende Alkylsubstiutenten sind und die anderen Substituenten, die nicht Alkyl sind, Wasserstoff sind.

Beispiele für bevorzugte alkylsubstituierte Phenole sind ortho-, meta- und para-Kresol, ortho-Ethylphenol, ortho-n- Butylphenol, ortho-sec.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-cyclohexyl-phenol, 2,6-Diethylphenol, 2,5-Diisopropylphenol, 2-Methyl-6-sec.-butylphenol, 3-tert.-Butylphenol, 2,6-Di-sec.-Butylphenol oder 2,6-Dicyclohexylphenol.

In einer bevorzugten Ausführungsform werden 2,6-Di-(C₁₋₁₂-alkyl)-phenole, bevozugt 2,6-Di-(C₁-₈-alkyl)-phenole und besonders bevorzugt 2,6-Di-(C₁₋₄-alkyl)-phenole in das erfindungsgemäße Verfahren eingesetzt.

Mit dem erfindungsgemäßen Verfahren bevorzugt hergestellte aromatische Amine sind 2,6-Di-(C₁-₈-alkyl)-aniline aus den entsprechenden 2,6-Di-(C₁₋₈-alkyl)-phenolen. Beispiele sind 2,6-Dimethylanilin (2,6-Xylidin), 2,6-Diethylanilin, 2-Methyl-6-ethylanilin, 2,6-Diisopropylanilin, 2-Isopropyl-6-methylanilin und 2-Isopropyl-6-ethylanilin.

Ein mit dem erfindungsgemäßen Verfahren besonders bevorzugt hergestelltes aromatische Amin ist 2,6-Dimethylanilin (2,6-Xylidin) durch Umsetzung von 2,6-Dimethylphenol.

Als weiterer Ausgangsstoff wird in das erfindungsgemäße Verfahren ein Amininierungsmittel eingesetzt.
Als Aminierungsmittel können sowohl Ammoniak als auch primäre oder sekundäre Amine, wie aliphatische oder cycloaliphatische oder aromatische Amine eingesetzt werden.

Das Aminierungsmittel ist bevorzugt eine Stickstoffverbindung der Formel II 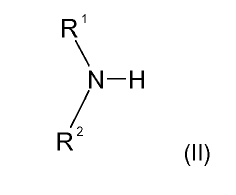 in der
- R¹, R²: jeweils zusammen oder unabhängig voneinander
Wasserstoff (H),
Alkyl, wie C₁₋₁₂-Alkyl, bevorzugt C₁₋₈-Alkyl, besonders bevorzugt bevorzugt C₁₋₄-Alkyl, insbesondere Methyl, Ethyl, n-Propyl, iso- Propyl, n-Butyl, iso-Butyl, sec.-Butyl und tert.-Butyl,
Cycloalkyl, wie C₃₋₁₂-Cycloalkyl, bevorzugt C₅- bis C₈-Cycloalkyl, besonders bevorzugt C₅- bis C₆-Cycloalkyl, insbesondere Cyclopentyl und Cyclohexyl,
Alkoxyalkyl, wie C₂₋₃₀-Alkoxyalkyl,
Dialkylaminoalkyl, wie C₃₋₃₀-Dialkylaminoalkyl,
Aryl, wie C₆ -C₁₄-Aryl, wie Phenyl, Naphthyl oder Anthracenyl;
Aralkyl, wie C₇₋₂₀-Aralkyl, bevorzugt C₇₋₁₂-Aralkyl wie z.B. Phenylmethyl, 1-Phenylethyl 2-Phenylethyl, 1-Phenylpropyl, 2-Phenylpropyl oder 3-Phenyl propyl.
oder gemeinsam -(CH₂)ⱼ-X-(CH₂)ₖ-bedeuten und
- X CH₂,: CHR¹⁰ Sauerstoff (O), Schwefel (S) oder NR¹⁰,
- R¹⁰: Wasserstoff (H), Alkyl, bevorzugt C₁₋₄-Alkyl, Alkylphenyl, bevorzugt C₇₋₄₀-Alkylphenyl,
- j, k: eine ganze Zahl von 1 bis 4,
bedeuten.

Besonders bevorzugt bedeuten
- R¹, R²: jeweils zusammen oder unabhängig voneinander
Wasserstoff (H),
Alkyl, wie C₁₋₁₂-Alkyl, bevorzugt C₁₋₈-Alkyl, besonders bevorzugt bevorzugt C₁₋₄-Alkyl, insbesondere Methyl, Ethyl, n-Propyl, iso- Propyl, n-Butyl, iso-Butyl, sec.-Butyl und tert.-Butyl,
Cycloalkyl, wie C₃₋₁₂-Cycloalkyl, bevorzugt C₅- bis C₈-Cycloalkyl, besonders bevorzugt C₅- bis C₆-Cycloalkyl, insbesondere Cyclopentyl und Cyclohexyl, oder
Aryl, wie C_{6 -}C₁₄-Aryl, wie Phenyl, Naphthyl oder Anthracenyl.

Die oben genannten Substituenten können ferner Substituenten tragen oder funktionelle Gruppen enthalten, welche sich unter den Bedingungen der hydrierenden Aminierung inert verhalten, beispielsweise, Alkyl-, Alkoxy-, Amino-, Alkylamino- oder Dialkylaminogruppen, oder auch gegebenenfalls unter den Bedingungen der hydrierenden Aminierung hydriert werden, beispielsweise CC-Doppel- oder Dreifachbindungen.

Ganz besonders bevorzugt wird das Aminierungsmittel ausgewählt aus der Gruppe bestehend aus Ammoniak, Monomethylamin, Dimethylamin, Monoethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Insbesondere bevorzugte Aminierungsmittel sind Ammoniak sowie Monomethylamin und Dimethylamin. Ganz besonders bevorzugt wird Ammoniak als Aminierungsmittel eingesetzt.

Als weiterer Einsatzstoff wird Wasserstoff in das erfindungsgemäße Verfahren eingesetzt.
Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Katalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Das erfindungsgemäße Verfahren wird in Gegenwart eines Katalysatorformkörpers durchgeführt.

Der Katalysatorformkörper ist dadurch gekennzeichnet, dass der Katalysatorformkörper eine Ringtablettenform aufweist.

Der Außendurchmesser D_{A} der Ringtablette liegt im Bereich von 3 bis 6 mm, bevorzugt im Bereich von 4 bis 6 mm und ganz besonders bevorzugt im Bereich von 5 bis 6 mm.

Der Innendurchmesser Dᵢ der Ringtablette liegt im Bereich von 2 bis 5 mm, bevorzugt im Bereich von 3 bis 5 mm und ganz besonders bevorzugt im Bereich von 3 bis 4 mm.

Die Höhe h (bzw. Dicke) der Ringtablette liegt im Bereich von 1 bis 4 mm, bevorzugt im Bereich von 2 bis 4 mm und ganz besonders bevorzugt im Bereich von 2,5 bis 3,5 mm.

Es ist auch möglich, dass der Formkörper eine von der Ringtablettenform abweichende Geometrie aufweist, die topologisch mit der Ringtablettenform äquivalent bzw. homöomorph ist.
Beispielsweise kann der Formkörper jede beliebige Geometrie, wie elliptisch, quadratisch, rechteckig, dreieckig, rautenförmig, sternförmig etc. aufweisen und mit einer Ausnehmung bzw. Aussparung (oder auch Loch) versehen sein, die ebenfalls jede beliebige Geometrie aufweisen kann.

Das Volumen des topologisch äquivalenten Formkörpers wird so gewählt, dass das Volumen des topologisch äquivalenten Formkörpers im Bereich des Volumen einer erfindungsgemäßen Ringtablette liegt (V = ((D_{A}-Dᵢ)^{2·}π / 4)·h).
Das Volumen der Aussparung bzw. Ausnehmung des topologisch äquivalenten Formkörpers wird entsprechend so gewählt, dass das Volumen der Aussparung bzw. Ausnehmung im Bereich des Volumens einer Aussparung oder Ausnehmung einer erfindungsgemäßen Ringtablette liegt (V = (Dᵢ^{2·}π / 4)·h).

In eine bevorzugten Ausführungsform sind die Abmessungen der Formkörper, sowie der topologisch äquivalenten Formkörper so bemessen, dass der größte Durchmesser des Formkörpers D_{A,max} größer ist als die Höhe des Formkörpers an seiner höchsten Stelle hₘₐₓ. Bevorzugt liegt das Verhältnis von D_{A,max} zu hₘₐₓ im Bereich von 1:0,1 bis 1:0,95, besonders bevorzugt im Bereich von 1:0,3 bis 1:0,9 und besonders bevorzugt im Bereich von 1:0,5 bis 1:0,8.

Wenn der Formkörper eine von der Ringform abweichende topologisch äquivalente Form aufweist, dann ist es weiterhin bevorzugt, dass die Aussparung bzw. die Ausnehmung so gestaltet ist, dass die Wandstärke des Formkörpers an seiner dünnsten Stelle bevorzugt mindestens 0,5 mm, besonders bevorzugt mindestens 1,0 mm und besonders bevorzugt mindestens 1,2 mm beträgt. Bevorzugt liegt die Wandstärke des topologisch äquivalenten Formkörpers bevorzugt im Bereich von 0,5 bis 4 mm, bevorzugt im Bereich von 1 bis 3 mm und ganz besonders bevorzugt im Bereich von 1,2 bis 2,5 mm. Beträgt die Wandstärke weniger als 0,5 mm, ist der Formkörper nicht mehr so gut mechanisch beanspruchbar, so dass der Formkörper brechen kann. Dies kann zu Verstopfungen des Katalysatorbetts führen.

Weiterhin ist es möglich, dass die Höhe h des Formköpers im Bereich des zuvor genannten Bereichs von 1 bis 4 mm, bevorzugt im Bereich von 2 bis 4 mm und ganz besonders bevorzugt im Bereich von 2,5 bis 3,5 mm variiert. Beispielsweise kann der Formkörper abgerundete Kanten und oder Ecken aufweisen (Torus-Form), so dass die Höhe von dem Rand der Katalysatorformkörpers nach innen zunehmen und/oder abnehmen kann.

Bevorzugt weist der Formköper jedoch eine Ringtabletten (bzw. Ringscheibenform) auf, da sich eine solche Form am einfachsten und wirtschaftlichsten herstellen lässt und zudem einen niedrigen Druckverlust in der Katalysatorschüttung verursacht.

Der Katalysatorformkörper enthält eine katalytisch aktive Masse und ggf. weitere Zusatzstoffe, wie Verformungshilfsmittel, Trägermaterialien oder weitere katalytisch aktive Bestandteile.

Die Angaben hinsichtlich der Zusammensetzung (in Gew.-%) beziehen sich auf die Gesamtmasse des Katalysatorformkörpers nach seiner letzten Temperung (Trocknung oder Kalzinierung) und vor der Behandlung des Katalysatorformkörpers mit Wasserstoff (Reduktion bzw. Aktivierung).
In dieser Form enthält der Katalysatorformkörper in der Regel weniger als 5 Gew.-% Wasser, bevorzugt weniger als 3,5 Gew.-% Wasser und besonders bevorzugt weniger als 1,5 Gew.-% Wasser.

Der Katalysatorformkörper enthält bevorzugt 50 Gew.-% und mehr einer katalytisch aktiven Masse.
Die katalytisch aktive Masse des Katalysatorformkörpers ist als die Summe der Massen der sauerstoffhaltigen Verbindungen von Zr, Pd und Pt nach der letzten Temperung des hergestellten Katalysatorformkörpers und vor der Behandlung (Reduktion bzw. Aktivierung) mit Wasserstoff definiert.

Besonders bevorzugt enthält der Katalysatorformkörper 75 Gew.-% und mehr, insbesondere bevorzugt 90 Gew.-% und mehr und ganz besonders bevorzugt 94 Gew.-% und mehr katalytisch aktive Masse.

In einer bevorzugten Ausführungsform enthält die katalytisch aktive Masse der im erfindungsgemäßen Verfahren eingesetzten Katalysatorformkörper 75 bis 99,8 Gew.-%, bevorzugt 90 bis 99,6 Gew.-%, besonders bevorzugt 94 bis 99,2 Gew.-% Zirkoniumdioxid (ZrO₂),
0,1 bis 12,5 Gew.-%, bevorzugt 0,2 bis 5 Gew.-%, besonders bevorzugt 0,2 bis 2,5 Gew.-% und ganz besonders bevorzugt 0,2-0,8 Gew.-% sauerstoffhaltige Verbindungen des Palladiums und
0,1 bis 12,5 Gew.-%, bevorzugt 0,2 bis 5 Gew.-%, besonders bevorzugt 0,2 bis 2,5 Gew.-% und ganz besonders bevorzugt 0,2-0,8 Gew.-% sauerstoffhaltige Verbindungen des Platins.

Der Katalysatorformkörper kann neben der katalytisch aktiven Masse weitere Zusatzstoffe enthalten, wie weitere katalytische Bestandteile, Formhilfsmittel oder Trägermaterialien.

Beispielsweise kann der Katalysatorformkörper als Zusatzstoff weitere katalytisch aktive Bestandteile enthalten.
Bevorzugt sind die die weiteren katalytisch aktiven Bestandteile Elemente der Gruppen 1, 2, 13 bis 15, sowie IB bis VIII B des Periodensystems, wobei die weiteren katalytisch aktiven Bestandteile, nach der letzten Temperung und vor der Behandlung mit Wasserstoff (Reduktion bzw. Aktivierung) in Abhängigkeit ihrer Oxidierbarkeit in der Regel entweder in Form ihrer Element (Oxidationsstufe 0) oder in Form ihrer sauerstoffhaltigen Verbindungen vorliegen.
Der Anteil weiterer katalytisch aktiver Bestandteile an den Zusatzstoffen bezogen auf den Katalysatorformkörper beträgt bevorzugt 0 bis 50 Gew.-%, bevorzugt 0,01 bis 25 Gew.-% und besonders bevorzugt 1 bis 10 Gew.-%.
In einer ganz besonders bevorzugten Ausführungsform enthalten die Katalysatorformkörper im Wesentlichen keine weiteren katalytisch aktiven Bestandteile.

Der Katalysatorformkörper kann weiterhin Trägermaterialien enthalten.
Als Trägermaterialien kommen beispielsweise Kohlenstoff, wie Graphit, Russ und/oder Aktivkohle, Aluminiumoxid (gamma, delta, theta, alpha, kappa, chi oder Mischungen daraus), Siliziumdioxid, Zeolithe, Alumosilicate oder deren Gemische in Betracht.
Der Anteil an Trägermaterialien bezogen auf den Katalysatorformkörper beträgt bevorzugt 0 bis 50 Gew.-%, bevorzugt 1 bis 25 Gew.-% und besonders bevorzugt 3 bis 10 Gew.-%.
In einer ganz besonders bevorzugten Ausführungsform enthalten die Katalysatorformkörper im Wesentlichen keine weiterenTrägermaterialien.

Der Katalysatorformkörper kann als Zusatzmittel Formhilfsmittel enthalten. Bevorzugte Formhilfsmittel sind Graphit oder Stearinsäure.
Der Anteil an Formhilfsmitteln bezogen auf den Katalysatorformkörper beträgt bevorzugt 0 bis 20 Gew.-%, bevorzugt 1 bis 15 Gew.-% und besonders bevorzugt 3 bis 10 Gew.-%.
In einer ganz besonders bevorzugten Ausführungsform enthalten die Katalysatorformkörper im Wesentlichen keine Formhilfsmittel.

Zur Herstellung der Katalysatorformkörper mit der genannten Geometrie und Zusammensetzung sind verschiedene Verfahrensweisen möglich.

In einer bevorzugten Ausführungsform wird ein Katalysatorformkörper mit erfindungsgemäßer Geometrie hergestellt, der Zr in Form von Zirkoniumdioxid (ZrO₂) enthält und welcher anschließend mit einer Lösung, die lösliche Verbindungen von Pd und Pt enthält, in Kontakt gebracht wird (Tränkung).

Zirkoniumdioxid kann in der monoklinen oder tetragonalen Modifikation eingesetzt werden. Bevorzugt wird ein Zirkoniumdioxid eingesetzt, dass zu 60 Gew.-% oder mehr in der monoklinen Modikation, besonders bevorzugt zu 75 Gew.-% oder mehr in der monoklinen Modifikation und ganz besonders bevorzugt zu 90% oder mehr in der monoklinen Modifikation vorliegt.

Vor der Verformung wird Zirkoniumdioxid üblicherweise konditioniert.
Die Konditionierung kann beispielsweise dadurch erfolgen, dass man Zirkoniumdioxid durch Vermahlen auf eine bestimmte Korngröße einstellt.
Die Einstellung der Korngrößen kann mittels des Fachmanns bekannten Verfahren, wie Mahlung oder Sprühtrocknung, erfolgen.
Bevorzugt wird Zirkoniumdioxid mit einer Korngröße von 0.1 µm bis 0,1 mm, besonders bevorzugt 0,5 µm bis 500 µm und ganz besonders bevorzugt 1 µm bis 100 µm eingesetzt.
Die Konditionierung kann auch das Vermischen von Zirkoniumdioxid mit einem Formhilfsmittel, bevorzugt Graphit oder Stearinsäure, insbesondere bevorzugt Graphit, oder ggf. weiteren Trägermaterialien beinhalten.

Ggf. können dem konditionierten Zirkoniumdioxid vor der Formgebung noch Lösungsmittel, Anteigmittel oder Porenbildner zugegeben werden.
Lösungsmittel und/oder Anteigmittel können beispielsweise zugegeben werden, um die Konsistenz des konditionierten Zirkoniumdioxids zu verändern. Dies kann ggf. vorteilhaft sein, wenn die Formgebung durch Extrusion erfolgen soll (siehe unten).

Als Lösungsmittel eignen sich z.B. acyclische oder cyclische Ether mit 2 bis 12 Kohlenstoffatomen, wie Diethylether, Di-n-propylether oder dessen Isomere, MTBE, THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art. Dem Wasser können sowohl Brönsted-Säuren als auch Brönstedt-Basen zugesetzt werden.
Der Anteil an Lösungsmittel liegt bevorzugt im Bereich von 0,5 bis 80 Gew.-%, weiter bevorzugt im Bereich von 1 bis 50 Gew.-%, weiter bevorzugt im Bereich von 1 bis 40 Gew.-%, und besonders bevorzugt im Bereich von 1 bis 30 Gew.-%, jeweils bezogen auf die Masse des eingesetzten konditionierten Zirkoniumdioxids.

Als Anteigmittel (= Anteigungsmittel) können alle dafür geeigneten Verbindungen verwendet werden. Vorzugsweise sind dies organische, insbesondere hydrophile Polymere wie beispielsweise Cellulose, Cellulosederivate, wie beispielsweise Methylcellulose, Stärke, wie beispielsweise Kartoffelstärke, Polyacrylate, Polymethacrylate, Polyvinylalkohol, Polyvinylpyrrolidon (PVP), Polyisobuten (PIB) oder Polytetrahydrofuran (PTHF). Der Anteil an Anteigmittel liegt bevorzugt im Bereich von 0,5 bis 80 Gew.-%, weiter bevorzugt im Bereich von 1 bis 50 Gew.-%, weiter bevorzugt im Bereich von 1 bis 40 Gew.-%, und besonders bevorzugt im Bereich von 1 bis 30 Gew.-%, jeweils bezogen auf die Masse des eingesetzten konditionierten Zirkoniumdioxids.

Als Porenbildner können im erfindungsgemäßen Verfahren sämtliche Verbindungen eingesetzt werden, die bezüglich des fertigen Formkörpers eine bestimmte Porengröße, eine bestimmte Porengrößenverteilung und/oder bestimmte Porenvolumina bereitstellt.
Bevorzugt werden als Porenbildner im erfindungsgemäßen Verfahren Polymere eingesetzt, die in Wasser oder in wässrigen Lösungsmittelgemischen dispergier-, suspendier- und/oder emulgierbar sind. Bevorzugte Polymere sind hierbei polymere Vinylverbindungen wie beispielsweise Polyalkylenoxide, wie Polyethylenoxide, Polystyrol, Polyacrylate, Polymethacrylate, Polyolefine, Polyamide und Polyester, Kohlenhydrate, wie Cellulose oder Cellulosederivate, wie beispielsweise Methylcellulose, oder Zucker oder Naturfasern. Weitere geeignete Porenbildner sind Pulp oder Graphit.
Bevorzugt sind auch organische saure Verbindungen, die durch Kalzinieren entfernen lassen. Zu nennen sind hier Carbonsäuren, insbesondere C₁₋₈-Carbonsäuren, wie beispielsweise Ameisensäure, Oxalsäure und/oder Zitronensäure. Ebenso ist es möglich, zwei oder mehr dieser saueren Verbindungen einzusetzen.
Werden Porenbildner eingesetzt, so liegt der Anteil an Porenbildner bevorzugt im Bereich von 0,5 bis 80 Gew.-%, bevorzugt im Bereich von 1 bis 50 Gew.-% und besonders bevorzugt im Bereich von 1 bis 30 Gew.-%, jeweils bezogen auf die die Masse des eingesetzten konditionierten Zirkoniumdioxids.
Sollte dies für die zu erzielende Porengrößenverteilung erwünscht sein, kann auch ein Gemisch aus zwei oder mehr Porenbildnern eingesetzt werden.

Die Porenbildner werden in einer besonders bevorzugten Ausführungsform des erfindungsgemäßen Verfahrens, wie untenstehend beschrieben, durch Kalzinieren unter Erhalt des porösen Formkörpers zu mindestens 90 Gew.-% entfernt.
Gemäß einer bevorzugten Ausführungsform des erfindungsgemäßen Verfahrens werden dabei Formkörper erhalten, die Porenvolumina, bestimmt gemäß DIN 66134, im Bereich von mindestens 0,1 ml/g, evorzugt im Bereich von 0,1 bis 0,5 ml/g und insbesondere bevorzugt im Bereich von mehr als 0,15 ml/g bis 0,35 ml/g aufweisen.
Die spezifische Oberfläche des erfindungsgemäßen Formkörpers, bestimmt gemäß DIN 66131 liegt bevorzugt bei mindestens 30 m²/g und insbesondere bei mindestens 50 m²/g.

Vor der Formgebung wird das konditionierte Zirkoniumdioxid mit den ggf. weiteren zugefügten Bestandteilen homogenisiert, z.B. für eine Dauer im Bereich von 10 bis 180 Minuten. Besonders bevorzugt werden zur Homogenisierung unter anderem Mischer, Kneter, Koller oder Extruder eingesetzt.
Die Homogenisierung wird bevorzugt bei Umgebungstemperatur oder bei erhöhten Temperaturen durchgeführt. Wenn ein Lösungsmittel zugeführt wird, so wird jedoch bevorzugt unterhalb des Siedepunkts des Lösungsmittels gearbeitet. Die Mischung aus konditioniertem Zirkoniumdioxid und ggf. weiteren Zusatzstoffen sowie ggf. weiteren zugefügten Bestandteilen wird solange homogenisiert bis eine einheitliche, durchmischte Masse entstanden ist.

Im Anschluss an die Homogenisierung erfolgt in der Regel die Formgebung des konditionierten Zirkoniumdioxids zu einem Formkörper mit erfindungsgemäßer Geometrie.

Die Formgebung erfolgt durch übliche Verfahren, wie sie beispielsweise in Ullmann's Encyclopedia of Industrial Chemistry, "Heterogeneous Catalysis and Solid Catalysts", Kapitel 5.2.2. (DOI: 10.1002/14356007.a05 313.pub2) oder in James T. Richardson, "Principles of Catalyst Development (Perspectives on Individual Differences)", Springer, Berlin, 1989, Kapitel 6.8, oder in B. Stiles, T. A Koch, "Catalyst Manufacture", M. Dekker, NewYork, 1995, Kapitel 9 beschrieben ist.
Hierbei erfolgt die Formgebung bevorzugt durch Kompaktierung oder Verpressung des konditionierten Zirkoniumdioxids in Formkörperformen mit erfindungsgemäßer Geometrie, z.B. durch Pelletierung bzw. Tablettierung .
Die Formgebung kann auch durch Extrusion des konditionierten Zirkoniumdioxids mit geeigneten Profildüsen erfolgen. Zur Extrusion werden dem konditionierten Zirkoniumdioxid in der Regel noch Lösungsmittel und/oder Anteigmittel zugegeben werden, damit das konditionierte Zirkoniumdioxid die richtige Konsistenz für die Weiterverarbeitung hat.

Bei der Herstellung der erfindungsgemäßen Formkörper durch Extrusion erfolgt nach dem Formgebungsprozess in der Regel ein Temperschritt (=Temperung), insbesondere ein Trocknungs- und/oder Kalzinierungsschritt, bei denen der Formkörper üblicherweise bei höheren Temperaturen temperiert wird. In der Regel wird der Trocknungs- und/oder Kalzinierungsschritt bei Temperaturen von 80 bis 600°, vorzugsweise 120 bis 450°C, besonders bevorzugt 350 bis 450°C durchgeführt.
Wenn die Herstellung der erfindungsgemäßen Formkörper durch Tablettierung erfolgt, so erfolgt bevorzugt im Anschluss an die Formgebung und vor der Tränkung in der Regel kein Temperschritt.

Die Tränkung der Formkörper mit Pd- und Pt-Verbindungen kann nach den üblichen Verfahren erfolgen (A. B. Stiles, Catalyst Manufacture - Laboratory and Commercial Preperations, Marcel Dekker, New York, 1983), beispielsweise durch Aufbringung von löslichen Verbindungen von Pd und Pt bzw. der weiteren katalytisch aktiven Bestandteile in einer oder mehreren Tränkstufen.
Als lösliche Verbindungen kommen in der Regel wasserlösliche Metallsalze, wie die Nitrate, Acetate oder Chloride von Pd und Pt bzw. der weiteren katalytisch aktiven Bestandteile in Betracht. Die Tränkung kann auch mit anderen geeigneten löslichen Verbindungen der entsprechenden Elemente erfolgen.
Im Anschluss wird der getränkte Formkörper in der Regel getrocknet und kalziniert. Die Trocknung erfolgt üblicherweise bei Temperaturen von 80 bis 200°C, vorzugsweise 100 bis 150°C.
Die Kalzinierung wird im Allgemeinen bei Temperaturen von 300 bis 600°C, vorzugsweise 350 bis 500°C, besonders bevorzugt 380 bis 480°C, durchgeführt.
Die Tränkung kann auch nach der sogenannten "incipient wetness-Methode" erfolgen, bei der der Katalysatorformkörper entsprechend seiner Wasseraufnahmekapazität maximal bis zur Sättigung mit der Tränklösung befeuchtet wird. Die Tränkung kann aber auch in überstehender Lösung erfolgen.
Bei mehrstufigen Tränkverfahren ist es zweckmäßig, zwischen einzelnen Tränkschritten zu trocknen und ggf. zu kalzinieren. Die mehrstufige Tränkung ist vorteilhaft dann anzuwenden, wenn der Formkörper in größerer Menge mit löslichen Verbindungen beaufschlagt werden soll.
Zur Aufbringung mehrerer Komponenten auf den Formkörper, kann beispielsweise die Tränkung gleichzeitig mit allen löslichen Verbindungen oder in beliebiger Reihenfolge der löslichen Verbindungen nacheinander erfolgen.

Die durch Imprägnierung erhaltenen Katalysatorformkörper enthalten nach der Kalzinierung die Komponenten der katalytisch aktiven Masse in Form eines Gemisches ihrer sauerstoffhaltigen Verbindungen, d.h. insbesondere als Oxide, Mischoxide und/oder Hydroxide. Die so hergestellten Katalysatorformkörper können als solche gelagert und gehandhabt werden.

Die Katalysatorformkörper, die in das erfindungsgemäße Verfahren eingesetzt werden, werden dadurch erhalten, dass man die Katalysatorformkörper, die wie voranstehend beschrieben durch Imprägnierung hergestellt wurden, nach der dem letzten Temperierungsschritt (Kalzinierung) reduziert bzw. aktiviert.

Dabei kann die Aktivierung bzw. Reduktion des Katalysatorformkörpers direkt im Reaktor unter den Bedingungen des erfindungsgemäßen Verfahrens erfolgen.

In einer bevorzugten Ausführungsform erfolgt die Reduktion bzw. Aktivierung der Katalysatorformkörper vor ihrem Einsatz in das erfindungsgemäße Verfahren (sogenannte Vorreduktion).
Die Reduktion des trockenen Katalysatorformkörpers kann bei erhöhter Temperatur in einem bewegten oder unbewegten Reduktionsofen durchgeführt werden.
Als Reduktionsmittel wird üblicherweise Wasserstoff oder ein Wasserstoff enthaltendes Gas eingesetzt.
Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, ggf. vermischt mit Frisch-Wasserstoff und ggf. nach Entfernen von Wasser durch Kondensation.
Die Reduktion des Katalysatorformkörpers erfolgt bevorzugt in einem Reaktor, in dem die Katalysatorformkörper als Festbett angeordnet sind. Besonders bevorzugt erfolgt die Reduktion des Katalysatorformkörpers in demselben Reaktor in dem die nachfolgende Umsetzung von aromatischem Alkohol mit Aminierungsmittel erfolgt.
Die Reduktion des Katalysatorformkörpers erfolgt in der Regel bei Reduktionstemperaturen von 50 bis 600°C, insbesondere von 100 bis 500°C, besonders bevorzugt von 150 bis 450°C.
Der Wasserstoffpartialdruck beträgt in der Regel von 0,1 bis 300 bar, insbesondere von 0,1 bis 100 bar, besonders bevorzugt von 0,1 bis 10 bar, wobei sich die Druckangaben hier und im Folgenden auf den absolut gemessenen Druck beziehen.
Die Dauer der Reduktion beträgt bevorzugt 1 bis 48 Stunden, und besonders bevorzugt 5 bis 15 Stunden.
Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden. Geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der aktivierte bzw. reduzierte Katalysatorformkörper kann nach der Reduktion unter inerten Bedingungen gehandhabt werden. Bevorzugt kann der aktivierte Katalysatorformkörper unter einem Inertgas wie Stickstoff gehandhabt und gelagert werden oder unter einer inerten Flüssigkeit, zum Beispiel einem Alkohol, Wasser, dem Produkt oder Edukt der jeweiligen Reaktion, für die der Katalysatorformkörper eingesetzt wird. Gegebenenfalls muss der Katalysatorformkörper vor Beginn der eigentlichen Reaktion dann von der inerten Flüssigkeit befreit werden.
Die Lagerung des Katalysatorformkörpers unter inerten Substanzen ermöglicht eine unkomplizierte und ungefährliche Handhabung und Lagerung des Katalysatorformkörpers.

Der Katalysatorformkörper kann nach der Reduktion aber auch mit einem Sauerstoff enthaltenden Gasstrom wie Luft oder einem Gemisch von Luft mit Stickstoff in Kontakt gebracht werden. Dadurch wird ein passivierter Katalysatorformkörper erhalten. Der passivierte Katalysatorformkörper weist im Allgemeinen eine schützende Oxidschicht auf. Durch diese schützende Oxidschicht wird die Handhabung und Lagerung des Katalysatorformkörpers vereinfacht, so dass beispielsweise der Einbau des passivierten Katalysatorformkörpers in den Reaktor vereinfacht wird. Passivierte Katalysatorformkörper werden vor Beginn der Reaktion in der Regel wie vorangehend beschrieben erneut aktiviert bzw. reduziert.

Die Herstellung der aromatischen Amine erfolgt durch Umsetzung des entsprechenden aromatischen Alkohols mit einem Aminierungsmittel in Gegenwart von Wasserstoff und eines Katalysatorformkörpers bei einer Temperatur von 60 bis 300°, bevorzugt in der Gasphase. Die Umsetzung kann aber auch in der Flüssigphase erfolgen.

Für die Synthese in der Gasphase wird der eingesetzte aromatische Alkohol gezielt, bevorzugt in einem Kreisgasstrom, verdampft und gasförmig dem Reaktor zugeführt. Das Kreisgas dient zum einen der Verdampfung des eingesetzten aromatischen Alkohols und zum anderen als Reaktionspartner für die Aminierung.

In der Kreisgasfahrweise werden die Ausgangsstoffe (aromatischer Alkohol, Wasserstoff und Aminierungsmittel) in einem Kreisgasstrom verdampft und gasförmig dem Reaktor zugeführt.
Die Edukte (aromatischer Alkohol und Aminierungsmittel) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer, bevorzugt zweier oder dreier, einzelner Rohrreaktoren bestehen. Optional ist hier vorteilhaft eine Zwischeneinspeisung von Feed (enthaltend das Edukt und/oder Ammoniak und/oder H₂) und/oder Kreisgas und/oder Reaktoraustrag aus einem nachgeschalteten Reaktor möglich.

Das Aminierungsmittel kann bezüglich der zu aminierenden alkoholischen Hydroxylgruppe in stöchiometrischen, unter- oder überstöchiometrischen Mengen eingesetzt werden.
Das Aminierungsmittel wird im Allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe eingesetzt.
Höhere Überschüsse an Aminierungsmittel sind möglich.

Bevorzugt wird eine Abgasmenge von 5 bis 800 Normkubikmeter/h, insbesondere 20 bis 300 Normkubikmeter/h, gefahren.

Das erfindungsgemäße Verfahren wird üblicherweise bei 0,1 bis 40 MPa (1 bis 400 bar), bevorzugt 0,1 bis 10 MPa, besonders bevorzugt 0,1 bis 0,5 MPa durchgeführt.
Die Temperaturen betragen im Allgemeinen 60 bis 300 °C, besonders 100 bis 290 °C, bevorzugt 120 bis 280 °C, besonders bevorzugt 160 bis 270 °C.
In einer besonders bevorzugten Ausführungsform wird das Verfahren in einer Anlage aus 2 hintereinandergeschalteten Reaktoren, insbesondere Rohrbündelreaktoren, ausgeführt oder in einem Reaktor mit 2 Temperaturzonen, wobei es bevorzugt ist, dass die Reaktortemperatur im zweiten Reaktor bzw. in der ersten Temperaturzone im Bereich von 5 bis 50°, bevorzugt im Bereich von 10 bis 40°C und besonders bevorzugt im Bereich von 15 bis 35°C über der Temperatur des ersten Reaktors bzw. der ersten Temperaturzone liegt.

Der Druck im Reaktionsgefäß, welcher sich aus der Summe der Partialdrücke des A-minierungsmittels, des aromatischen Alkohols und der gebildeten Reaktionsprodukte sowie ggf. des mit verwendeten Lösungsmittels bei den angegebenen Temperaturen ergibt, wird zweckmäßigerweise durch Aufpressen von Wasserstoff auf den gewünschten Reaktionsdruck erhöht.

Die Katalysatorbelastung liegt im Allgemeinen im Bereich von 0,01 bis 2, bevorzugt 0,05 bis 0,5 kg aromatischer Alkohol pro Liter Katalysator (Schüttvolumen) und Stunde. Es ist dabei sowohl eine Anströmung des Katalysatorfestbetts von oben als auch von unten möglich. Den erforderlichen Gasstrom erhält man bevorzugt durch eine Kreisgasfahrweise.

Die Kreisgasmenge liegt bevorzugt im Bereich von 40 bis 2000 m³ (bei Betriebsdruck) / [m³ Katalysator (Schüttvolumen) h], insbesondere im Bereich von 700 bis 1700 m³ (bei Betriebsdruck) / [m³ Katalysator (Schüttvolumen) • h].

Das Kreisgas enthält bevorzugt mindestens 10, besonders 50 bis 100, ganz besonders 60 bis 90, Vol.% H₂.

Der Wasserstoff wird der Reaktion im Allgemeinen in einer Menge von 5 bis 400 l, bevorzugt in einer Menge von 5 bis 100 l pro Mol aromatischer Alkoholkomponente zugeführt, wobei die Literangaben jeweils auf Normalbedingungen umgerechnet wurden (S.T.P.).

Beim kontinuierlichen Arbeiten in der Gasphase kann das überschüssige Aminierungsmittel zusammen mit dem Wasserstoff im Kreis geführt werden.

Das im Zuge der Umsetzung gebildete Reaktionswasser (jeweils ein Mol pro Mol umgesetzte Alkoholgruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z.B. durch eine fraktionierende Rektifikation. Das überschüssige Aminierungsmittel und der Wasserstoff werden vorteilhaft wieder in die Reaktionszone zurückgeführt. Das gleiche gilt für die eventuell nicht vollständig umgesetzten aromatischen Alkohol.

Die jeweiligen Reinprodukte können aus den Rohwaren durch Rektifikation nach den bekannten Methoden erhalten werden. Die Reinprodukte fallen als Azeotrope mit Wasser an oder können in Anlehnung an die Patentanmeldungen EP-A-1 312 599 und EP-A-1 312 600 durch eine Flüssig-flüssig-Extraktion mit konzentrierter Natronlauge entwässert werden. Diese Entwässerung kann vor oder nach der Reindestillation erfolgen. Eine destillative Entwässerung in Gegenwart eines Schleppmittels nach bekannten Methoden ist auch möglich.

Für den Fall, dass die Rohware oder das aromatische Amin in der Rohware kaum oder nicht mit Wasser mischbar sind, ist eine Entwässerung durch eine Trennung der organischen und der wässrigen Phase mit bekannten Methoden auch möglich. Gemäß der gelehrten Vorgehensweise in EP-A-1 312 599 und in EP-A-1 312 600 können von der separierten organischen Phase in einem Schritt eine oder mehrere Leichtsiederfraktionen von dem aminhaltigen Gemisch destillativ abgetrennt werden. In einem weiteren Schritt besteht die Möglichkeit, eine oder mehrere Schwersiederfraktionen von dem aminhaltigen Gemisch destillativ abzutrennen. In einem folgenden Destillationsschritt kann aus der Mischung das im wesentlichen wasserfreie Amin als Sumpfabzug oder Seitenabzug der Kolonne in reiner Form erhalten werden, welches gegebenenfalls einer weiteren Aufreinigung oder Auftrennung unterworfen wird.

Diese Einzelschritte zur Aufreinigung des aromatischen Amins können gegebenenfalls auch in einer einzigen Kolonne batchweise oder kontinuierlich durchgeführt werden, wobei eine Abtrennung der Leichtsieder über den Kopfabzug und/oder den Seitenabzug des Verstärkungsteils der Kolonne, eine Abtrennung der Schwersiederfraktionen über den Sumpfabzug der Destillationskolonne und die Abtrennung des reinen Amins über den Seitenabzug im Abtriebsteil der Kolonne erfolgen kann.

In einer besonders bevorzugten Variante kommt als kontinuierliche Destillationskolonne eine Trennwandkolonne zum Einsatz.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Die erfindungsgemäßen Katalysatorformkörper ermöglichen ein verbessertes wirtschaftliches Verfahren zur Herstellung von primären aromatischen Aminen aus Phenolen. Insbesondere werden bessere Ausbeuten, Raum-Zeit-Ausbeuten (RZA) und Selektivitäten bei einem solchen Verfahren erreicht. Darüber hinaus wird die Bildung von typischen Nebenprodukten, wie Cyclohexanolen, Dicyclohexylamin, Diphenylaminen u.a., die nur schwer vom Wertprodukt abzutrennen sind, verringert. Des Weiteren wird ein Katalysator mit einer höheren Aktivität zur Verfügung gestellt, der seine Aktivität über einen möglichst langen Zeitraum aufrecht erhält. Der verwendete Katalysator führt zu einer Verbesserung der Standzeiten und ermöglicht damit weniger häufige Regenerierungen, wodurch die Abstellungen zum Katalysatorwechsel im technischen Betrieb verringert werden können.

Die Erfindung wird durch nachfolgende Beispiele erläutert.

### Beispiele

### Herstellung des Katalysators:

### Formkörper A (Vergleichsbeispiel):

1,5 kg ZrO₂ Pulver werden mit 5% Graphit vermischt und anschließend zu Ringtabletten von 7 (D_{A')} x 3 (Dᵢ) x 3 (h) mm verpresst. Der Pressdruck der Tablettenpresse wird so eingestellt, dass für die hergestellten Tabletten eine Wasseraufnahme von 0,18-0,28 ml/g erhalten wird. Von den Ringtabletten wird die genaue Wasseraufnahme bestimmt und die Konzentratrion der Tränklösungen für eine Tränkung auf Wasseraufnahme mit 0,46% PdO und 0,43% PtO berechnet. Anschließend werden die Ringtabletten in einer Imprägniertrommel mit einer Lösung von Palladiumnitrat und Platinnitrat getränkt, dass die Lösung auf die Ringtabletten gesprüht wird. Die Ringe werden dann bei 120°C 4 h getrocknet und danach bei 520°C für 2h kalziniert. Der so hergestellte Katalysatorformkörper enthielt 0,46 Gew.-% PdO (berechnet als Metall der Oxidationsstufe II) und 0,43 Gew.-% PtO (berechnet als Metall der Oxidationsstufe II) auf ZrO₂.

### Formkörper B (erfindungsgemäß):

1,5 kg ZrO₂ Pulver werden mit 5% Graphit vermischt und anschließend zu Ringtabletten von 5,5 (D_{A}) x 3 (Dᵢ) x 3 (h) mm verpresst. Der Pressdruck der Tablettenpresse wird so eingestellt, dass für die hergestellten Tabletten eine Wasseraufnahme von 0,18-0,28 ml/g erhalten wird. Von den Ringtabletten wird die genaue Wasseraufnahme bestimmt und die Konzentratrion der Tränklösungen für eine Tränkung auf Wasseraufnahme mit 0,46% PdO und 0,43% PtO berechnet Anschließend werden die Ringtabletten in einer Imprägniertrommel mit einer Lösung von Palladiumnitrat und Platinnitrat derart getränkt, dass die Lösung auf die Ringtabletten gesprüht wird. Die Ringe werden dann bei 120°C 4 h getrocknet und danach bei 520°C für 2 h kalziniert. Der so hergestellte Katalysatorformkörper enthält dann 0,46 Gew.-% PdO (berechnet als Metall der Oxidationsstufe II) und 0,43 Gew.-% PtO (berechnet als Metall der Oxidationsstufe II) auf ZrO₂.

### Testung der Katalysatoren:

Die Experimente wurden in einer kontinuierlich betriebenen Gasphasenapparatur mit ölbeheiztem Reaktor (30x2x750 mm) bei Normaldruck durchgeführt. Dazu wurde geschmolzenes 2,6-Dimethylphenol (DMP) vor dem Reaktoreintritt mit Wasserstoff und Ammoniak vereinigt und nach einer Mischstrecke auf den oberen Teil des Reaktors geführt. Der obere Teil des Reaktors war mit 250 mL V2A-Stahlringen gefüllt (D = 3mm), darunter befand sich der entsprechende Katalysatorformkörper (A oder B) (100 mL), darunter wiederum 50 mL V2A-Stahlringe (D = 6mm). Nach Austritt aus dem Reaktor wurde der Reaktionsaustrag in einen Abscheider geführt, in dem der flüssige Austrag gesammelt wurde.

In Tabelle 1 sind die Ergebnisse der geschwindigkeitsbestimmenden ersten Stufe der Xylidinherstellung zusammengestellt. Die Ofentemperatur wurde jeweils auf 200°C eingestellt. Das Molverhältnis von Ammoniak zu Wasserstoff wurde bei 1,55:1 gehalten, das Molverhältnis von Ammoniak zu 2,6-Dimethylphenol (DMP) bei 6,60:1. Im ersten Zeitabschnitt der Experimente wurde die Katalysatorbelastung auf 0,153 kg/L_{Kat} h eingestellt (0-950 h), danach wurde die Belastung schrittweise erhöht (0,176 kg/L_{Kat} h; 950-1150 h und 0,204 kg/L_{Kat} h; 1150-1330 h).

Die Ergebnisse zeigen, dass besonders der Katalysator mit den 5,5 x 3 x 3 mm Formkörper sehr aktiv ist und nur sehr langsam desaktiviert, selbst nach 1330 h liegt der Rest-DMP-Gehalt noch bei weniger 2%, wohingegen bei dem ansonsten identischen Katalysator mit 7 x 3 x 3 mm Formkörper 6,3% Rest-DMP-gefunden werden. Auch der Anteil an leichtsiedenden Nebenprodukten ist mit dem erfindungsgemäßen Katalysator geringer. Mit den erfindungsgemäßen Katalysatorformkörpern kann ein höherer Umsatz von 2,6-DMP erzielt werden. Zudem ist die Ausbeute an Wertprodukten (2,6-Xylidin und DMCH-a, DMCH-on und DMCH-ol) erhöht und die Menge an unerwünschten Leichtsiedern geringer.

**Tabelle 1:**

| **Eintrag** | **Katalysator** | **Belastung [kg/Lh]** | **Laufzeit [h]** | **2,6-DMP [GC Fl.%]** | **Leichtsieder [GC Fl.%]** | **DMCH -a, -on, -ol [GC FI.%]** | **2,6-Xylidin [GC FI.%]** |
|---|---|---|---|---|---|---|---|
| **1** | 7x3x3 | 0,153 | 950 | 2,1 | 3,4 | 38,6 | 55,1 |
| **2** | 7x3x3 | 0,176 | 1150 | 3,3 | 3,2 | 39,7 | 53,7 |
| **3** | 7x3x3 | 0,204 | 1330 | 6,3 | 2,7 | 39,6 | 51,3 |
| | | | | | | | |
| **4** | 5,5x3x3 | 0,153 | 950 | 0,5 | 3,0 | 45,4 | 51,0 |
| **5** | 5,5x3x3 | 0,176 | 1150 | 1,0 | 2,4 | 45,9 | 50,6 |
| **6** | 5,5x3x3 | 0,204 | 1330 | 1,7 | 2,1 | 47,0 | 49,2 |

| | | | | | | | |
|---|---|---|---|---|---|---|---|
| 2,6-DMP = 2,6-Dimethylphenol DMCH-a = 2,6-Dimethylcyclohexylamin DMCH-on = 2,6-Dimethylcyclohexanon DMCH-ol = 2,6-Dimethylcyclohexanol | | | | | | | |

Die o.g. Gehalte in Gew.-% werden wie folgt gaschromatografisch bestimmt:
Trennsäule : DB WAX (Polyethylenglycol)
Länge (m): 30
Filmdicke (µm): 0,5
Innendurchmesser (mm): 0,25
Trägergas: Helium
Vordruck (bar): 1,0
Split (ml/Min.): 100
Septumspülung (ml/Min.): 4
Ofentemperatur (°C): 80
Vorheizzeit (Min.): 3
Rate (°C/Min.): 5
Ofentemperatur (°C): 240
Nachheizzeit (Min.): 30
Injektortemperatur (°C): 250
Detektortemperatur (°C): 260
Injektion: HP 7673-Autosampler
Injetionsvolumen (mikrol): 0,2
Detektortyp: FID
GC-Methode: GC-FI.% - Methode

## Patentansprüche

1. Verfahren zur Herstellung eines aromatischen Amins durch Umsetzung eines entsprechenden aromatischen Alkohols mit einem Aminierungsmittel ausgewählt aus der Gruppe bestehend aus Ammoniak, primärer Amine und sekundärer Amine,
in Gegenwart von Wasserstoff und
eines Katalysatorformkörpers,
bei einer Temperatur von 60 bis 300°,
**dadurch gekennzeichnet, dass** der Katalysatorformkörper Zr, Pd und Pt enthält und eine Ringtablettenform mit einem Außendurchmesser im Bereich von 2 bis 6 mm und einer Höhe im Bereich von 1 bis 4 mm und einem Innendurchmesser von 1 bis 5 mm oder eine topologisch äquivalente Form mit gleichem Volumen aufweist.

2. Verfahren nach Anspruch 1, **dadurch gekennzeichnet, dass** der Katalysatorformkörper eine aktive Masse enthält, die vor der Reduktion der aktiven Masse mit Wasserstoff
75 bis 99,8 Gew.% sauerstoffhaltige Verbindungen des Zirkoniums,
0,01 bis 12,5 Gew.-% sauerstoffhaltige Verbindungen des Palladiums und
0,01 bis 12,5 Gew.-% sauerstoffhaltige Verbindungen des Platins
enthält.

3. Verfahren nach mindestens einem der Ansprüche 2 bis 3, **dadurch gekennzeichnet, dass** der Katalysatorformkörper 50 Gew.-% und mehr aktive Masse enthält, bezogen auf den Katalysatorformkörper.

4. Verfahren nach mindestens einem der Ansprüche 1 bis 3 **dadurch gekennzeichnet, dass** der Katalysatorformkörper optional 0 bis 50 Gew.-% eines oder mehrer Zusatzmittel enthält.

5. Verfahren nach Anspruch 4, durch gekennzeichnet, dass das Zusatzmittel Graphit ist.

6. Verfahren nach mindestens einem der Ansprüche 1 bis 5, **dadurch gekennzeichnet, dass** als aromatischer Alkohol ein Phenol der Formel (I) 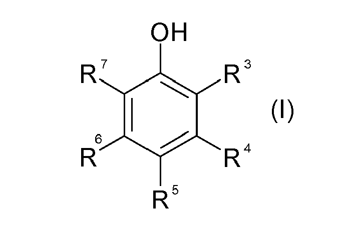 eingesetzt wird, in der R³, R⁴, R⁵, R⁶ und R⁷ jeweils zusammen oder unabhängig voneinander Wasserstoff (H), C₁-C₁₂-Alkyl, oder C₃-C₁₂-Cycloalkyl bedeuten.

7. Verfahren nach Anspruch 6, **dadurch gekennzeichnet, dass** das eingesetzte Phenol 2,6-Dimethylphenol ist.

8. Verfahren nach einem der Ansprüche 1 bis 7, **dadurch gekennzeichnet, dass** das Aminierungsmittel ein Aminierungsmittel der Formel (II) ist, 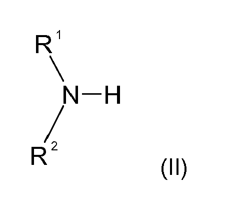 in der R¹, R² jeweils zusammen oder unabhängig voneinander Wasserstoff (H), Alkyl, Cycloalkyl, Alkoxyalkyl, Dialkylaminoalkyl, Aryl, Aralkyl, oder gemeinsam - (CH₂)ⱼ-X-(CH₂)ₖ- bedeuten und X bedeutet CH₂, CHR¹⁰ Sauerstoff (O), Schwefel (S) oder NR¹⁰ und R¹⁰ bedeutet Wasserstoff (H), Alkyl oder Alkylphenyl und j, k bedeuten eine ganze Zahl von 1 bis 5.

9. Katalysatorformkörper enthaltend Zr, Pd und Pt, **dadurch gekennzeichnet dass** der Katalysatorformkörper eine Ringtablettenform, mit einem Außendurchmesser im Bereich von 3 bis 6 mm und einer Höhe im Bereich von 1 bis 4 mm und einem Innendurchmesser von 2 bis 5 mm oder eine topologisch äquivalente Form aufweist.

10. Katalysatorformkörper gemäß Anspruch 9, **dadurch gekennzeichnet, dass** der Katalysatorformkörper eine aktive Masse enthält, die vor der Reduktion der aktiven Masse mit Wasserstoff
75 bis 99,8 Gew.% Zirkoniumdioxid (ZrO₂),
0,01 bis 12,5 Gew.-% sauerstoffhaltige Verbindungen des Palladiums und
0,01 bis 12,5 Gew.-% sauerstoffhaltige Verbindungen des Platins
enthält.

11. Katalysatorformkörper gemäß mindestens einem der Ansprüche 9 bis 10, **dadurch gekennzeichnet, dass** der Katalysatorformkörper 50 Gew.-% und mehr aktive Masse aufweist und optional 0 bis 50 Gew.-% Zusatzstoffe enthält.

12. Katalysatorformkörper gemäß mindestens einem der Ansprüche 9 bis 11, **dadurch gekennzeichnet, dass** zunächst ein Katalysatorformkörper mit erfindungsgemäßer Geometrie hergestellt, der Zr in Form von Zirkoniumdioxid (ZrO₂) enthält und welcher anschließend mit einer Lösung, die lösliche Verbindungen von Pd und Pt enthält, in Kontakt gebracht wird (Tränkung).
